(19)

Europäisches Patentamt

European Patent Office

Office européen des brevets

(11) **EP 4 494 588 A1**

(12) **EUROPEAN PATENT APPLICATION**
published in accordance with Art. 153(4) EPC

(43) Date of publication:
**22.01.2025 Bulletin 2025/04**

(21) Application number: **22940742.4**

(22) Date of filing: **11.10.2022**

(51) International Patent Classification (IPC):
**A61B 18/14** (2006.01)     **A61B 18/12** (2006.01)

(86) International application number:
**PCT/CN2022/124543**

(87) International publication number:
**WO 2023/213048 (09.11.2023 Gazette 2023/45)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **05.05.2022 CN 202210556942**
**05.05.2022 CN 202210482325**

(71) Applicant: **Innolcon Medical Technology (Suzhou) Co., Ltd.**
**Jiangsu 215000 (CN)**

(72) Inventors:
• **YAO, Longyang**
**Suzhou, Jiangsu 215000 (CN)**
• **WANG, Fuyuan**
**Suzhou, Jiangsu 215000 (CN)**
• **WEI, Dalun**
**Suzhou, Jiangsu 215000 (CN)**
• **LIU, Zhenzhong**
**Suzhou, Jiangsu 215000 (CN)**
• **LUO, Wei**
**Suzhou, Jiangsu 215000 (CN)**

(74) Representative: **Otten, Roth, Dobler & Partner mbB Patentanwälte**
**Großtobeler Straße 39**
**88276 Berg / Ravensburg (DE)**

(54) **METHOD FOR CALCULATING IN REAL TIME CONTROL PARAMETERS OF HIGH-FREQUENCY ELECTROTOME OUTPUT SYSTEM, GENERATOR, AND ELECTROTOM**

(57)     The present disclosure provides a method for real-time calculation of control parameters of a high-frequency electrosurgical knife output system, a generator and an electrosurgical knife. The control parameters include but are not limited to output voltage and output current. Whether the two electrodes (104, 105) of a bipolar tool (103) effectively clamp tissue is determined by an initial voltage and current phase difference value and an initial impedance value (303). The output power is adjusted according to a predetermined power change trajectory, until the tissue impedance value reaches a target change characteristic (307). The output energy is adjusted according to a predetermined voltage change trajectory, until the tissue impedance value reaches an end impedance threshold (308). That the tissue impedance value is not lower than the impedance threshold within a predetermined time is determined (308). Parameters such as the impedance and phase difference of the fundamental frequency and the second harmonic are comprehensively considered in the method, more accurate actual voltage and current phase difference values and actual tissue impedance values are obtained through calculation, and more precise control of the tissue closure process on this basis is achieved.

FIG 2

EP 4 494 588 A1

**Description**

The present application is a divisional application of the Chinese patent application No. 202210482325.2 filed on May 5, 2022, and entitled "TISSUE CLOSURE METHOD WITH HIGH-FREQUENCY ELECTROSURGICAL KNIFE, SYSTEM, GENERATOR AND HIGH-FREQUENCY ELECTROSURGICAL KNIFE".

**FIELD OF THE INVENTION**

The present disclosure relates to the field of surgical instruments, and in particular to a method for real-time calculation of control parameters of a high-frequency electrosurgical knife output system, a generator and an electrosurgical knife.

**BACKGROUND OF THE INVENTION**

The high-frequency electrosurgical knife is a medical instrument that closes tissues by electrical energy, and generally includes a distally mounted end actuator configured for bipolar or monopolar operation. During bipolar operation, current passes through the tissue through an active electrode and a return electrode of the end actuator. During monopolar operation, current passes through the tissue through an active electrode of the end actuator and a return electrode separately disposed on the patient's body. The heat generated by the current flowing through the tissue can form a hemostatic seal within and/or between tissues, and thus can be used for tissue closure.

The frequency of the high-frequency electrosurgical knife's output signal is generally higher than 100 kHz, which can avoid stimulating human muscles and nerves. The generator controls the surgical effect by controlling the power and power factor of the output signal. Generally speaking, the higher the power factor, the better the electrocoagulation effect, and the higher the output peak voltage. The high-frequency electrosurgical knife system has multiple sets of voltage and current sensors, which sample the output voltage and current signals in real time. The controller processes the sampled voltage and current signals to obtain control parameters such as impedance and phase, and controls the real-time output power based on these control parameters.

During actual surgery, due to the complex surgical environment, including the influence of temperature, blood, body fluid penetration, surrounding tissue, tool status and other factors, the circuit and tissue have changing parasitic inductance and capacitance. The actual output feedback signal may deviate from the ideal sinusoidal signal and contain relatively complex high-frequency harmonics, resulting in the actual tissue impedance value or actual voltage and current phase difference directly calculated by a single FFT algorithm being very inaccurate.

**SUMMARY OF THE INVENTION**

The purpose of the present disclosure is to overcome the shortcomings of the prior art and provide a method for real-time calculation of control parameters of a high-frequency electrosurgical knife output system, a generator and an electrosurgical knife.

The purpose of the present disclosure is achieved through the following solutions.

A method for real-time calculation of control parameters of a high-frequency electrosurgical knife output system, comprising the following steps:

Sampling output parameters of the output system in real time; and

Calculating an actual voltage and current phase difference value $\vartheta$ and an actual tissue impedance value Z of the high-frequency electrosurgical knife output system in real time.

Preferably, the step of calculating the actual voltage and current phase difference value $\vartheta$ of the high-frequency electrosurgical knife output system in real time specifically includes:

Calculating a reference phase difference value $\vartheta_{ref}$ by using a mean value calculation algorithm or a zero-crossing detection algorithm;

Calculating a fundamental frequency phase difference value $\vartheta_1$ and a second harmonic phase difference value $\vartheta_2$ of the doubled frequency of the output signal by a Goertzel algorithm or an FFT algorithm; and

Obtaining the actual voltage and current phase difference value $\vartheta$ by taking a weighted average value of the fundamental frequency phase difference value $\vartheta_1$ and the second harmonic phase difference value $\vartheta_2$ using formula 1, wherein weight coefficients are calculated according to the fundamental frequency phase difference value $\vartheta_1$ and the second harmonic phase difference value $\vartheta_2$ using formula 2 and formula 3,

$$\vartheta = w_{\vartheta 1}\vartheta_1 + w_{\vartheta 2}\vartheta_2 \qquad (\text{formula 1}),$$

$$w_{\vartheta 1} = f_{\vartheta 1}\left(\frac{\vartheta_1}{\vartheta_{ref}}\right) \qquad \text{(formula 2),}$$

$$w_{\vartheta 2} = f_{\vartheta 2}\left(\frac{\vartheta_2}{\vartheta_{ref}}\right) \qquad \text{(formula 3),}$$

wherein $w_{\vartheta 1}$ and $w_{\vartheta 2}$ are the weight coefficients.

Preferably, the step of calculating the reference phase difference value $\vartheta_{ref}$ by using the mean value calculation algorithm specifically includes:

Providing real-time sampled output parameters of the output system;

Calculating a real-time voltage effective value $V_{rms}$, a real-time current effective value $I_{rms}$, and a real-time effective power value $P_{rms}$ according to the output parameters; and

Calculating the reference phase difference value $\vartheta_{ref}$ according to formula 4,

$$\cos(\vartheta_{ref}) = \frac{P_{rms}}{V_{rms} \times I_{rms}} \qquad \text{(formula 4).}$$

Preferably, the step of calculating the reference phase difference value $\vartheta_{ref}$ by the zero-crossing detection algorithm specifically includes:

Providing real-time sampled output parameters of the output system, from which the phase difference $\vartheta_V$ at zero-crossing point of the sampled voltage and the phase difference $\vartheta_I$ at zero-crossing point of the sampled current are obtained; and

Calculating the reference phase difference value $\vartheta_{ref}$ according to formula 5,

$$\vartheta_{ref} = \vartheta_V - \vartheta_I \qquad \text{(formula 5).}$$

Preferably, the step of calculating the actual tissue impedance value Z of the high-frequency electrosurgical knife output system in real time specifically includes:

Calculating a reference impedance value $Z_{ref}$ by using an effective value algorithm;

Calculating a fundamental frequency impedance value $Z_1$ and a second harmonic impedance value $Z_2$ of the doubled frequency of the output signal by the Goertzel algorithm or the FFT algorithm; and

Obtaining the actual tissue impedance value Z by taking a weighted average value of the fundamental frequency impedance value $Z_1$ and the second harmonic impedance value $Z_2$ using formula 6, wherein weight coefficients are calculated according to the fundamental frequency impedance value $Z_1$ and the second harmonic impedance value $Z_2$ using formula 7 and formula 8,

$$Z = w_{Z1}Z_1 + w_{Z2}Z_2 \qquad \text{(formula 6),}$$

$$w_{Z1} = f_{Z1}\left(\frac{Z_1}{Z_{ref}}\right) \qquad \text{(formula 7),}$$

$$w_{Z2} = f_{Z2}\left(\frac{Z_2}{Z_{ref}}\right) \qquad \text{(formula 8),}$$

wherein $w_{Z1}$ and $w_{Z2}$ are the weight coefficients.

Preferably, the step of calculating the reference impedance value $Z_{ref}$ by using the effective value algorithm specifically includes:

Providing real-time sampled output parameters of the output system;

Calculating an effective value $V_{rms}$ of the real-time voltage and an effective value $I_{rms}$ of the real-time current according to the output parameters; and

Calculating the reference impedance value $Z_{ref}$ according to formula 9,

$$Z_{ref} = \frac{U_{rms}}{I_{rms}} \qquad \text{(formula 9).}$$

Preferably, it can be used to calculate the initial voltage and current phase difference value $\vartheta_0$ and the initial tissue impedance value $Z_0$.

The present disclosure discloses a generator of the high-frequency electrosurgical knife, comprising a control chip, a power generation and output circuit, a sampling circuit, and an interface circuit. The control chip has a control circuit therein, and the control circuit is configured to use the above-mentioned method for real-time calculation of control parameters of the high-frequency electrosurgical knife output system.

The present disclosure also discloses a high-frequency electrosurgical knife, comprising a surgical tool and the generator as described above.

Preferably, the surgical tool includes a bipolar tool or a monopolar tool.

The beneficial effects of the present disclosure are mainly reflected in that, parameters such as the impedance and phase difference of the fundamental frequency and the second harmonic are comprehensively considered in the invention, more accurate actual voltage and current phase difference values and actual tissue impedance values are obtained through calculation, and more precise control of the tissue closure process on this basis is achieved.

**BRIEF DESCRIPTION OF THE DRAWINGS**

The technical solution of the present disclosure is further described below in conjunction with the accompanying drawings.

FIG 1 is a system diagram of the high-frequency electrosurgical knife according to a preferred embodiment of the present disclosure.

FIG 2 is a functional block diagram of the high-frequency electrosurgical knife system according to a preferred embodiment of the present disclosure.

FIG 3 is a control flow chart of the tissue closure process according to a preferred embodiment of the present disclosure.

FIG 4 is a flow chart of the first stage of an implementation scheme of a preferred embodiment of the present disclosure.

FIG 5 is a flow chart of the second stage of an implementation scheme of a preferred embodiment of the present disclosure.

FIG 6 is a flow chart of the third stage of an implementation scheme of a preferred embodiment of the present disclosure.

FIG 7 is a flow chart of the fourth stage of an implementation scheme of a preferred embodiment of the present disclosure.

FIG 8 is a diagram of an output signal of a preferred embodiment of the present disclosure.

FIG 9 is a diagram showing the output power and voltage changes during the closing process of a preferred embodiment of the present disclosure.

**DETAILED DESCRIPTION OF THE EMBODIMENTS**

The present disclosure will be described in detail below in conjunction with the specific embodiments shown in the accompanying drawings. However, these embodiments are not limited to the present disclosure, and any structural, methodological, or functional changes made by a person skilled in the art based on these embodiments are all included in the protection scope of the present disclosure.

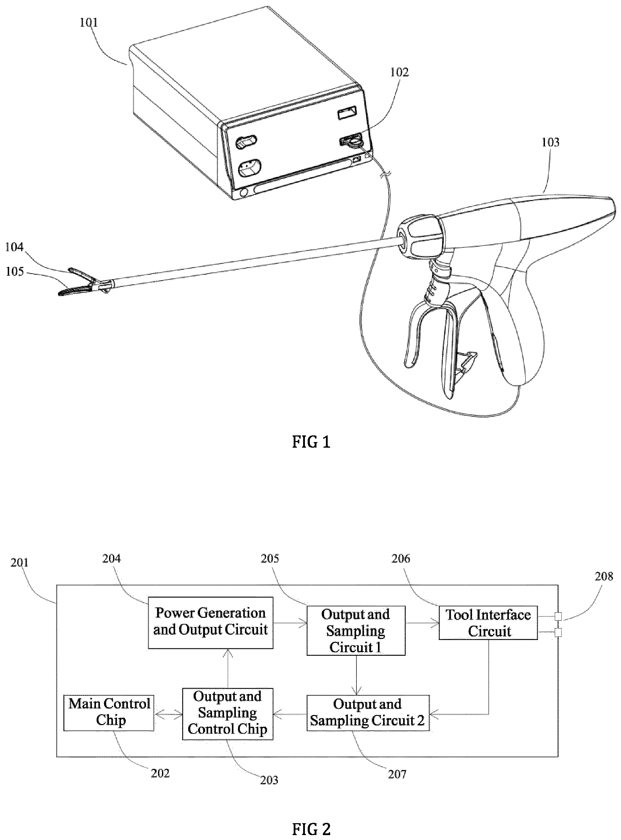

As shown in FIG 1, the present disclosure discloses a diagram of a high-frequency electrosurgical knife system, wherein 101 refers to the generator of the high-frequency electrosurgical knife, which is used to generate and control energy output to a tissue site, 102 refers to a socket for the surgical tool of an advanced bipolar mode; 103 refers to a bipolar tool, including a plug, a connecting wire and a tool head; and 104 and 105 refer to upper and lower electrodes at the front end of the tool head for clamping tissues. The energy output by the generator is finally transmitted to the tissue site via the connecting wire and the conductor connecting the two electrodes inside the tool head.

FIG 2 is a functional block diagram of the high-frequency electrosurgical knife system disclosed in the present disclosure, wherein 201 refers to a high-frequency electrosurgical knife host. The main control chip 202 can be a chip such as ARM, DSP, FPGA, ASIC, CPU, GPU, etc., and the system software control logic and algorithm mainly run in the main control chip. The main control chip 202 can receive the sampling signal implemented by the output and sampling control chip 203, and calculate at least one control parameter required by the algorithm according to the sampling signal, wherein the control parameter can be the tissue impedance value and the voltage and current phase difference value corresponding to the fundamental frequency or second harmonic of the output signal, and generate at least one control parameter according to the algorithm, wherein the control parameter can be the output power, output voltage, output current and other parameters. The output and sampling control chip 203 receives the control parameters sent by the main control chip, and performs real-time control on the power generation, and the output circuit and the output sampling circuit. Of course, the function of the output and sampling control chip 203 can also be realized by the main control chip 202. The power generation and output circuit 204 is configured to generate a real-time high-frequency alternative current (AC) signal according to the control signal of the sampling control chip 203, and output the high-frequency AC signal to the surgical tissue site via the tool interface circuit 206, the tool interface 208 and the connected tool. The output and sampling circuits 205 and 207 include at least one sensor, which can be a voltage or current sensor, and can sample the real-time output signal to obtain at least one set of output parameters, which can be output voltage and output current. The output sampling signal sends the sampled signal to the output and sampling control chip 203 to complete the closed-loop control of the entire system.

The output and sampling control chip 203 samples the real-time output voltage and current signals. Preferably, the output high-frequency signal is 450~550 kHz, and the sampling circuit samples 64 or 128 points per signal cycle and sends the sampling points to the output and sampling control chip 203.

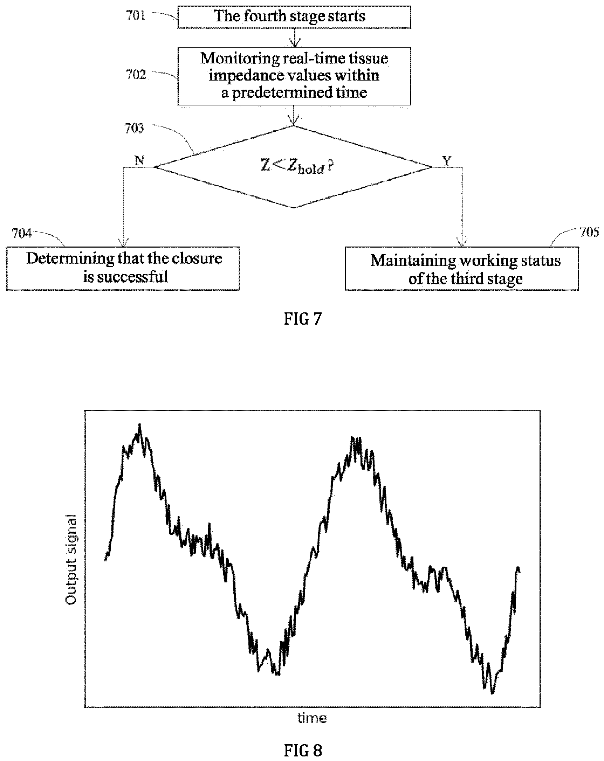

In one embodiment of the present disclosure, the output signal is shown in FIG 8. The output signal contains relatively complex high-order harmonics, and the calculation of tissue impedance and voltage and current phase difference using a single algorithm is not accurate enough. Therefore, the present disclosure proposes an optimized calculation method, which comprehensively considers parameters such as impedance and phase difference of the fundamental frequency and the second harmonic, and can obtain a more accurate and real-time calculation of the actual voltage and current phase difference $\vartheta$ and actual tissue impedance $Z$ of the high-frequency electrosurgical knife output system, thereby achieving more precise control of the closing process.

The specific explanation is as follows.

First, the reference phase difference value $\vartheta_{ref}$ and reference impedance value $Z_{ref}$ are calculated using the first algorithm, which may be a mean value calculation algorithm, a zero-crossing detection algorithm, or an effective value algorithm, wherein the reference phase value $\vartheta_{ref}$ is calculated using the mean value calculation algorithm or the zero-crossing detection algorithm, and the reference impedance value $Z_{ref}$ is calculated using the effective value algorithm.

Then, the second algorithm is used to calculate the fundamental frequency phase value $\vartheta_1$ and the second harmonic phase value $\vartheta_2$ of the doubled frequency of the output signal, the fundamental frequency impedance value $Z_1$ and the second harmonic impedance value $Z_2$ of the doubled frequency of the output signal. The second algorithm may be a Goertzel algorithm or an FFT algorithm.

The actual voltage and current phase difference value $\vartheta$ is obtained according to the weighted average value of the fundamental frequency phase value $\vartheta_1$ and the second harmonic phase value $\vartheta_2$, and the actual tissue impedance value Z is obtained according to the weighted average value of the fundamental frequency impedance value $Z_1$ and the second harmonic impedance value $Z_2$.

Specifically, based on the sampling signal, the effective value of the voltage $U_{rms}$ and the effective value of the current $I_{rms}$ can be calculated first, and the algorithms for the calculation are:

$$U_{rms} = \sqrt{\frac{\sum_1^N U_t^2}{N}} \ ,$$

$$I_{rms} = \sqrt{\frac{\sum_1^N I_t^2}{N}} \ .$$

And then the reference impedance value $Z_{ref}$ can be calculated according to the following formula:

$$Z_{ref} = \frac{U_{rms}}{I_{rms}} \ .$$

According to the zero-crossing time point $t_u$ of the voltage signal and the zero-crossing time point $t_I$ of the voltage signal of the sampling signal, the reference phase difference value $\vartheta_{ref}$ can be calculated. The algorithm is as follows:

$$\vartheta_{ref} = 2\pi * \frac{t_u - t_I}{T} \ ,$$

wherein T is the period of the output high-frequency signal.

Alternatively, the reference phase difference value $\vartheta_{ref}$ is calculated by a mean value calculation algorithm, specifically including:

Providing real-time sampled output parameters of the output system;

Calculating an effective value $V_{rms}$ of the real-time voltage, an effective value $I_{rms}$ of the real-time current, and an effective value $P_{rms}$ of the real-time power;

Calculating and obtaining the reference phase difference value $\vartheta_{ref}$ according to the following formula:

$$\cos\left(\vartheta_{ref}\right) = \frac{P_{rms}}{V_{rms} \times I_{rms}} \ .$$

Alternatively, the reference phase difference value $\vartheta_{ref}$ is calculated by a zero-crossing detection algorithm, specifically including:

Providing real-time sampled output parameters of the output system, from which the phase difference $\vartheta_V$ at zero-crossing point of the sampled voltage and the phase difference $\vartheta_I$ at zero-crossing point of the sampled current are obtained;

Calculating and obtaining the reference phase difference value $\vartheta_{ref}$ according to the following formula:

$$\vartheta_{ref} = \vartheta_V - \vartheta_I \ .$$

Based on the FFT algorithm or the Goertzel algorithm, the voltage peak value U1 and the phase difference value $\vartheta_{v1}$, the current peak value I1 and the phase difference value $\vartheta_{I1}$ of the fundamental frequency of the real-time output signal, as well as the voltage peak value U2 and the phase difference value $\vartheta_{v2}$, the current peak value I2 and the phase difference value $\vartheta_{I2}$ of the second harmonic are calculated.

According to the peak voltage Up1 and peak current Ip1 of the fundamental frequency, the fundamental frequency impedance value $Z_1$ and the voltage and current phase difference value $\vartheta_1$ of the fundamental frequency can be calculated according to the following formulas:

$$Z_1 = {U_1}/{I_1} \ ,$$

$$\vartheta_1 = \vartheta_{v1} - \vartheta_{I1} \ .$$

According to the peak voltage Up2 and peak current Ip2 of the second harmonic, the fundamental frequency signal impedance value $Z_2$ and the voltage and current phase difference value $\vartheta_2$ of the fundamental frequency can be calculated according to the following formulas:

$$Z_2 = {U_2}/{I_2} \ ,$$

$$\vartheta_2 = \vartheta_{v2} - \vartheta_{I2} \ .$$

The actual tissue impedance value Z can be obtained by taking the weighted average of $Z_1$ and $Z_2$ according to the following formula:

$$Z = w_{Z1} Z_1 + w_{Z2} Z_2 \ .$$

In the formula, $w_{Z1}$ and $w_{Z2}$ are weight coefficients, which are obtained by a mapping function of the ratio of $Z_1$ and $Z_2$ to the reference phase difference value $Z_{ref}$, and can be expressed as:

$$w_{Z1} = f_{Z1}\left(\frac{Z_1}{Z_{ref}}\right) \ ,$$

$$w_{Z2} = f_{Z2}\left(\frac{Z_2}{Z_{ref}}\right) \ .$$

The actual phase difference $\vartheta$ can be obtained by taking the weighted average of $\vartheta_1$ and $\vartheta_2$ using the following

formula:

$$\vartheta = w_{\vartheta 1}\vartheta_1 + w_{\vartheta 2}\vartheta_2 \ .$$

In the formula, $w_{\vartheta 1}$ and $w_{\vartheta 2}$ are weight coefficients, which are obtained by a mapping function of the ratio of $\vartheta_1$ and $\vartheta_2$ to the reference phase difference value $\vartheta_{ref}$, and can be expressed as:

$$w_{\vartheta 1} = f_{\vartheta 1}\left(\frac{\vartheta_1}{\vartheta_{ref}}\right) \ ,$$

$$w_{\vartheta 2} = f_{\vartheta 2}\left(\frac{\vartheta_2}{\vartheta_{ref}}\right) \ .$$

In this way, the actual tissue impedance value $Z$ and the actual voltage and current phase difference value $\vartheta$ can be obtained, and the output signal can be controlled in real time based on the result.

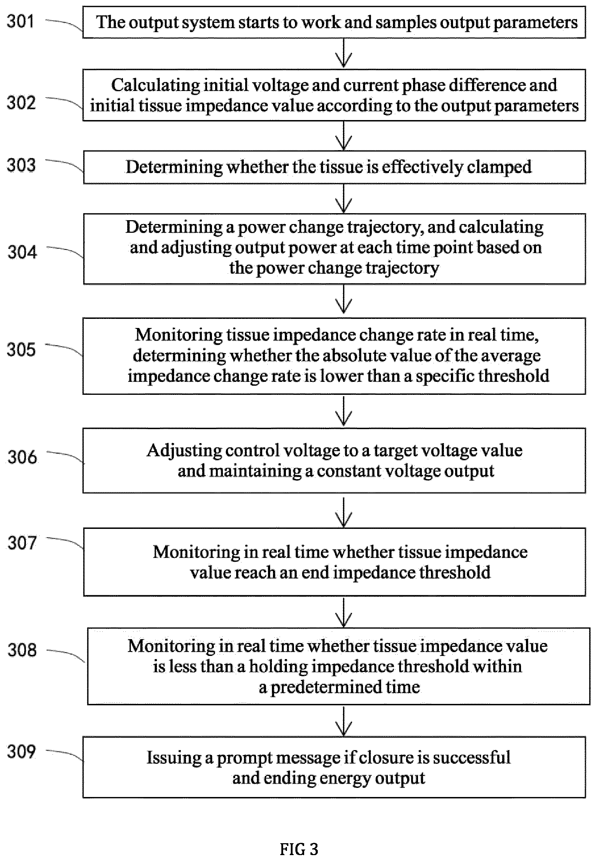

FIG 3 is a preferred tissue closure process control flow chart of the present disclosure, which is divided into four stages.

In the first stage, when the doctor presses the manual switch on the knife or steps on the corresponding foot pedal, the tissue closure process begins. In step 301, the high-frequency electrosurgical knife output system starts to output a small signal and performs sampling and calculation of the initial voltage and current phase difference $\vartheta_0$ and the initial tissue impedance value $Z_0$ in step 302. The small signal can be a high-frequency AC signal of 1~10 W, and the duration can be 10~300 ms. The initial voltage and current phase difference $\vartheta_0$ and the initial tissue impedance value $Z_0$ are the average phase value and the average tissue impedance value in the period of time, respectively. In a preferred embodiment of the present disclosure, the signal power is 4 W, the duration is 200 ms, and the frequency is 450 kHz.

Step 303 is the closing process, in which the algorithm determines whether the tool effectively clamps the tissue based on the initial voltage and current phase difference value $\vartheta_0$ and the initial tissue impedance value $Z_0$.

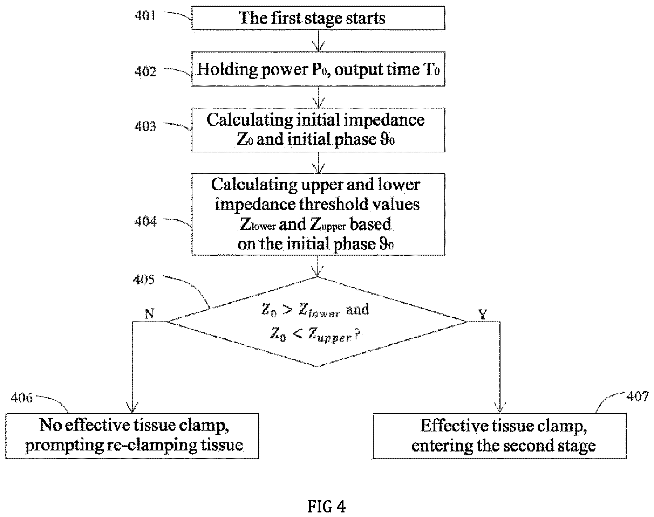

As shown in FIG 4, in a preferred embodiment of the present disclosure, the algorithm for determining effective tissue clamping is as follows:

Configuring a lookup table; and
Searching the lookup table, and obtaining an upper impedance threshold value $Z_{upper}$ and a lower impedance threshold value $Z_{lower}$ are obtained based on the initial voltage and current phase difference value $\vartheta_0$ and the lookup table, which is step 404.

In step 405, the relationship between the initial tissue impedance value $Z_0$ and the upper impedance threshold $Z_{upper}$ and the lower impedance threshold $Z_{lower}$ is compared.

In step 407, when the initial tissue impedance value $Z_0$ is between the upper impedance threshold $Z_{upper}$ and the lower impedance threshold $Z_{lower}$, it indicates that the front edge of the tool has effectively clamped the tissue, and then the second stage of the closing process can be entered.

Step 406 is divided into two cases. When the impedance value $Z_0$ is lower than the lower impedance threshold $Z_{lower}$, it means that there is too little tissue between the two electrodes of the tool or there is liquid between the electrodes. When the impedance value $Z_0$ is higher than the upper impedance threshold $Z_{upper}$, it means that there is too much tissue between the two electrodes of the tool or the circuit is open.

The second stage of the tissue closure process of the present disclosure is shown in step 304 and step 305 of FIG 3. The purpose of this stage is to denature and fuse the tissue.

At the beginning of this stage, as in step 304, firstly, a power change trajectory is determined by an algorithm according to the initial voltage and current phase difference $\vartheta_0$ and the initial tissue impedance $Z_0$. The trajectory may be a linear function, a quadratic function or other polynomial functions. At least one parameter of the trajectory can be determined according to the initial voltage and current phase difference $\vartheta_0$ and the initial tissue impedance $Z_0$.

The function of the power change trajectory is determined in advance based on the actual test results. For example, in a preferred embodiment, when the trajectory is a linear function, the parameters of the corresponding function can be a constant power value (including the initial power) and the rising slope of the linear trajectory, which is calculated from the initial voltage and current phase difference $\vartheta_0$ and the initial tissue impedance value $Z_0$. The calculation formula is, for example, *rising_slope = a \* $Z_0$ + b*, wherein *a* and *b* are fixed values. It is well known to those skilled in the art that there can be many calculation formulas, and the preferred embodiment is only an example. Other calculation formulas are also within the scope of the present disclosure. The second stage will calculate and adjust the output power at each time point according to the power change trajectory.

In the second stage, the average change rate of tissue impedance value may be monitored in real time, which corresponds to step 305. When the absolute value of the average impedance change rate is lower than a specific threshold, it means that the tissue has been completely fused and the impedance no longer changes. The impedance value will show a decreasing trend in the process. However, due to the actual complex surgical environment, including the influence of factors such as temperature, blood, body fluid penetration, surrounding tissues, and tool status, the vascular closure process may involve relatively complex biological and physical changes, and the impedance value will fluctuate. Directly using the real-time impedance value or the average impedance value for judgment will result in large errors. Actual studies have shown that the absolute value of the average impedance change rate is a relatively good characteristic for fusion judgment.

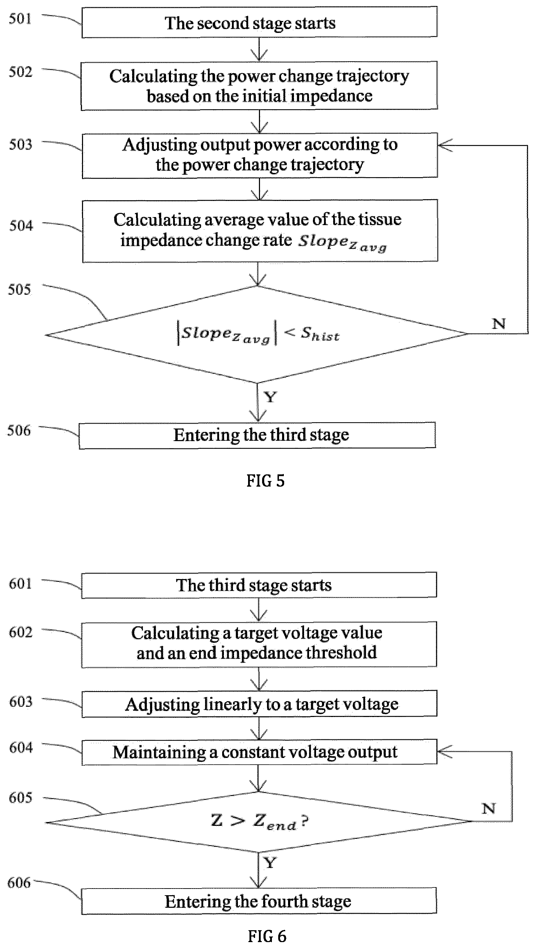

As shown in FIG 5, a preferred embodiment of the present disclosure includes:

Step 502, determining a power change trajectory according to the initial voltage and current phase difference value $\vartheta_0$ and the initial tissue impedance value $Z_0$;

Step 503, calculating and adjusting the output power at each time point based on the power change trajectory;

Step 504, monitoring the tissue impedance value change rate in real time and calculating an average value; and

Step 505, determining whether the absolute value of the average value of the impedance change rate is less than a specific threshold $S_{hist}$. If so, it indicates that the tissue has been completely fused, and proceeding to step 506.

The tissue impedance value change rate $Slope_z$ is calculated by the following formula, wherein $Z[t + 1]$ and $Z[t]$ are the impedances at two adjacent moments respectively:

$$Slope_Z[t + 1] = Z[t + 1] - Z[t] \ .$$

The mean value algorithm for the tissue impedance value change rate can use a slope average, a weighted average or an exponential average over a period of time. A preferred algorithm of the present disclosure uses the exponential average of a continuous time series, which has a very high computational efficiency and only needs to save the slope average of the previous moment. The algorithm is as follows, wherein $\alpha$ is the exponential average factor, which can be used to adjust the averaging degree, and the larger the value of $\alpha$, the more significant the averaging effect:

$$Slope_Z[t]_{avg} = \alpha * Slope_Z[t - 1]_{avg} + (1 - \alpha) * Slope_Z[t] \ .$$

When the tissue fusion is completed, the third stage of tissue closure will be entered, corresponding to step 306 and step 307 of FIG 3. In this stage, the high pressure of the front edge of the tool is mainly used to make the fused tissue in the second stage further completely fuse together, and make the fused tissue dry and solidify to form a firm closed area. As the tissue is completely fused, the gap between the two electrodes at the front edge of the tool will become smaller and smaller. This process requires controlling the appropriate voltage value so that the tissue can be dried and solidified relatively stably, and will not be broken down due to too small a gap, thereby improving the closing effect while preventing tissue breakdown and adhesion, and improving the tool life.

As shown in FIG 6, a preferred embodiment of the present disclosure includes:

Step 602, calculating a target voltage value and an end impedance threshold;

Step 603, adjusting the control voltage to the target voltage value;

Step 604, maintaining a constant voltage output; and

Step 605, monitoring in real time whether the tissue impedance value reaches the end impedance threshold; when the tissue impedance value is greater than the end impedance threshold, proceeding to step 606.

In the third stage, a target voltage value $U_{target}$ is first calculated based on the minimum impedance value in the second stage $Z_{min}$, and then the current voltage value is linearly adjusted to the target voltage $U_{target}$ within a predetermined time and then maintained at a constant voltage.

The formula for calculating the target voltage value $U_{target}$ may be a function, and the function may be a linear function or a polynomial function. In a preferred embodiment of the present disclosure, the algorithm is a linear function, and the calculation formula is as follows:

$$U_{target} = a * Z_{min} + b \ ,$$

wherein $a$ and $b$ are constants.

It is well known to those skilled in the art that there may be many calculation formulas, and the preferred embodiment is only an example, and other calculation formulas are also within the scope of the present disclosure.

At the beginning of the third stage, the end impedance threshold $Z_{end}$ is calculated according to the initial impedance value $Z_0$ and the minimum impedance value calculated in the second stage $Z_{min}$. The calculation formula can be a function, which can be a sum function or a weighted average function. In a preferred embodiment of the present disclosure, the algorithm is a weighted average function, and the calculation formula is as follows, wherein $\beta$ and $\gamma$ are weighted coefficients:

$$Z_{\text{end}} = \beta * Z_0 + \gamma * Z_{\min} \ .$$

In step 307, it is monitored whether the actual tissue impedance value reaches the end impedance threshold $Z_{\text{end}}$. When the actual tissue impedance value reaches or exceeds the end impedance threshold $Z_{\text{end}}$, the fourth stage is entered.

The fourth stage is shown in step 308 and step 309 of FIG 3. In this stage, whether the actual tissue impedance value is less than a holding impedance threshold within a predetermined time is monitored in real time in step 308; step 309, when the actual tissue impedance value is not less than the holding impedance threshold within the predetermined time, a prompt message for successful tissue closure is issued and the energy output of the high-frequency electrosurgical knife output system is terminated.

As shown in FIG 7, a preferred embodiment of the present disclosure includes:

Step 702, monitoring the real-time tissue impedance value within a predetermined time;
Step 703, determining simultaneously whether the real-time tissue impedance value is less than a holding impedance threshold $Z_{\text{hold}}$, and the formula for calculating the holding impedance threshold $Z_{\text{hold}}$ is as follows:

$$Z_{\text{hold}} = Z_{\text{end}} - Z_{\text{fall}} \ ,$$

wherein $Z_{\text{fall}}$ is a preset fixed impedance drop bias.
Step 704, determining that the closure is successful when the real-time tissue impedance value is not less than the holding impedance threshold $Z_{\text{hold}}$ within the predetermined time; and
Step 705, maintaining the working status of the third stage when the real-time tissue impedance value is less than the impedance threshold $Z_{\text{hold}}$ within the predetermined time.

In step 309, according to a preferred embodiment of the present disclosure, the energy output will be automatically stopped and an end prompt will be issued, and the prompt may include sound, flashing light, etc.

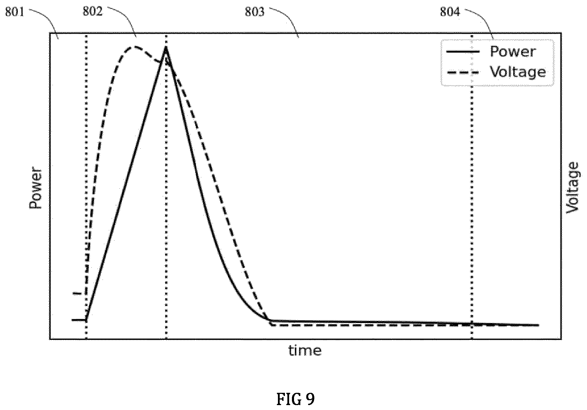

In a preferred implementation scheme of the present disclosure, the changes in output power and output voltage during a tissue closure process are shown in FIG 9. It can be seen from the figure that in the first stage 801, the power and the voltage are basically unchanged, which is used to test the initial impedance and phase. In the second stage 802, both the power value and the voltage value increase, and this stage is used to denature and fuse the tissue, and the tissue impedance gradually decreases. In the third stage 803, the voltage is first adjusted to the target voltage according to a predetermined trajectory, and then a constant voltage output is maintained. In the fourth stage 804, a constant voltage is maintained. In the third and fourth stages, the impedance gradually increases as the tissue is dried, and the output power gradually decreases.

The present disclosure also discloses a high-frequency electrosurgical tissue closure system, comprising:

An effective tissue clamping judgment unit including a sensor, which is configured to sample the output parameters of the high-frequency electrosurgical knife when the high-frequency electrosurgical knife starts to work, calculate the initial voltage and current phase difference value $\vartheta_0$ and the initial tissue impedance value $Z_0$ according to the output parameters, and determine whether the tissue is effectively clamped according to the initial voltage and current phase difference value $\vartheta_0$ and the initial tissue impedance value $Z_0$;
A tissue complete fusing judgment unit, which is configured to determine a power change trajectory according to the initial voltage and current phase difference value $\vartheta_0$ and the initial tissue impedance value $Z_0$, calculate and adjust the output power at each time point based on the power change trajectory, monitor the tissue impedance value change rate in real time, and determine whether the absolute value of the average value of the tissue impedance value change rate is less than a specific threshold value, and if so, it indicates that the tissue has been completely fused;
An output voltage adjustment unit, which is configured to adjust the control voltage to the target voltage value and maintain a constant voltage output, and monitor in real time whether the actual tissue impedance value reaches the end impedance threshold; and
A tissue closure success judgment unit, which is configured to monitor in real time whether the actual tissue impedance value is less than the impedance holding threshold within a predetermined time; when the actual tissue impedance value is not less than the impedance holding threshold within the predetermined time, a prompt message for successful tissue closure is issued and the energy output of the high-frequency electrosurgical knife output system is terminated.

The present disclosure also discloses a generator for the high-frequency electrosurgical knife, including a control chip, a power generation and output circuit, an adoption circuit, and an interface circuit, wherein the control chip has a control circuit therein, and the control circuit is configured to use the tissue closure method for the high-frequency electrosurgical knife as described above.

The present disclosure also discloses a high-frequency electrosurgical knife, including a bipolar tool and the generator as described above.

It should be understood that although this specification is described according to the above embodiments, not every embodiment contains only one independent solution. This description of the specification is only for the sake of clarity. Those skilled in the art should regard the specification as a whole. The solutions in each embodiment may also be appropriately combined to form other embodiments that can be understood by those skilled in the art.

The series of detailed descriptions above are only specific descriptions of feasible embodiments of the present disclosure. They are not intended to limit the scope of the present disclosure. Any equivalent embodiments or changes that do not deviate from the spirit of the present disclosure should be included in the scope of the present disclosure.

## Claims

1. A method for real-time calculation of control parameters of a high-frequency electrosurgical knife output system, comprising steps of:

   sampling output parameters of output system in real time; and
   calculating an actual voltage and current phase difference value $\vartheta$ and an actual tissue impedance value Z of the high-frequency electrosurgical knife output system in real time.

2. The method according to claim 1, wherein the step of calculating the actual voltage and current phase difference value $\vartheta$ of the high-frequency electrosurgical knife output system in real time comprises:

   calculating a reference phase difference value $\vartheta_{ref}$ by using a mean value calculation algorithm or a zero-crossing detection algorithm;
   calculating a fundamental frequency phase difference value $\vartheta_1$ and a second harmonic phase difference value $\vartheta_2$ of a doubled frequency of the output signal by a Goertzel algorithm or an FFT algorithm; and
   obtaining the actual voltage and current phase difference value $\vartheta$ by taking a weighted average value of the fundamental frequency phase difference value $\vartheta_1$ and the second harmonic phase difference value $\vartheta_2$ using formula 1, wherein weight coefficients are calculated according to the fundamental frequency phase difference value $\vartheta_1$ and the second harmonic phase difference value $\vartheta_2$ using formula 2 and formula 3,

   $$\vartheta = w_{\vartheta 1}\vartheta_1 + w_{\vartheta 2}\vartheta_2 \qquad \text{(formula 1)},$$

   $$w_{\vartheta 1} = f_{\vartheta 1}\left(\frac{\vartheta_1}{\vartheta_{ref}}\right) \qquad \text{(formula 2)},$$

   $$w_{\vartheta 2} = f_{\vartheta 2}\left(\frac{\vartheta_2}{\vartheta_{ref}}\right) \qquad \text{(formula 3)},$$

   wherein $w_{\vartheta 1}$ and $w_{\vartheta 2}$ are the weight coefficients.

3. The method according to claim 2, wherein the step of calculating the reference phase difference value $\vartheta_{ref}$ by using the mean value calculation algorithm comprises:

   providing real-time sampled output parameters of the output system;
   calculating a real-time voltage effective value $V_{rms}$, a real time current effective value $I_{rms}$, and a real-time effective power value $P_{rms}$ according to the output parameters; and
   calculating the reference phase difference value $\vartheta_{ref}$ according to formula 4,

$$\cos(\vartheta_{ref}) = \frac{P_{rms}}{V_{rms} \times I_{rms}} \qquad \text{(formula 4)}.$$

4. The method according to claim 2, wherein the step of calculating the reference phase difference value $\vartheta_{ref}$ by the zero-crossing detection algorithm comprises:

   providing real-time sampled output parameters of the output system, from which the phase difference $\vartheta_V$ at zero-crossing point of the sampled voltage and the phase difference $\vartheta_I$ at zero-crossing point of the sampled current are obtained;
   calculating the reference phase difference value $\vartheta_{ref}$ according to formula 5,

$$\vartheta_{ref} = \vartheta_V - \vartheta_I \qquad \text{(formula 5)}.$$

5. The method according to claim 1, wherein the step of calculating the actual tissue impedance value Z of the high-frequency electrosurgical knife output system in real time comprises:

   calculating a reference impedance value $Z_{ref}$ by using an effective value algorithm;
   calculating a fundamental frequency impedance value $Z_1$ and a second harmonic impedance value $Z_2$ of the doubled frequency of the output signal by the Goertzel algorithm or the FFT algorithm; and
   obtaining the actual tissue impedance value Z by taking a weighted average value of the fundamental frequency impedance value $Z_1$ and the second harmonic impedance value $Z_2$ using formula 6, wherein weight coefficients are calculated according to the fundamental frequency impedance value $Z_1$ and the second harmonic impedance value $Z_2$ using formula 7 and formula 8,

$$Z = w_{Z1}Z_1 + w_{Z2}Z_2 \qquad \text{(formula 6)},$$

$$w_{Z1} = f_{Z1}\left(\frac{Z_1}{Z_{ref}}\right) \qquad \text{(formula 7)},$$

$$w_{Z2} = f_{Z2}\left(\frac{Z_2}{Z_{ref}}\right) \qquad \text{(formula 8)},$$

   wherein $w_{Z1}$ and $w_{Z2}$ are the weight coefficients.

6. The method according to claim 5, wherein the step of calculating the reference impedance value $Z_{ref}$ by using the effective value algorithm comprises:

   providing real-time sampled output parameters of the output system;
   calculating an effective value $V_{rms}$ the real-time voltage and an effective value $I_{rms}$ of the real-time current according to the output parameters; and
   calculating the reference impedance value $Z_{ref}$ according to formula 9,

$$Z_{ref} = \frac{U_{rms}}{I_{rms}} \qquad \text{(formula 9)}.$$

7. The method according to claim 1, which is used to calculate the initial voltage and current phase difference value $\vartheta_0$ and the initial tissue impedance value $Z_0$.

8. A generator of a high-frequency electrosurgical knife, comprising a control chip, a power generation and output circuit, a sampling circuit, and an interface circuit, wherein the control chip has a control circuit therein, and the control circuit is configured to use the method for real-time calculation of control parameters of the high-frequency electrosurgical knife output system according to any one of claims 1 to 7.

9. A high-frequency electrosurgical knife, comprising a surgical tool and the generator according to claim 9.

**10.** The high-frequency electrosurgical knife according to claim 9, wherein the surgical tool is a bipolar tool or a monopolar tool.

FIG 1

FIG 2

301 — The output system starts to work and samples output parameters

302 — Calculating initial voltage and current phase difference and initial tissue impedance value according to the output parameters

303 — Determining whether the tissue is effectively clamped

304 — Determining a power change trajectory, and calculating and adjusting output power at each time point based on the power change trajectory

305 — Monitoring tissue impedance change rate in real time, determining whether the absolute value of the average impedance change rate is lower than a specific threshold

306 — Adjusting control voltage to a target voltage value and maintaining a constant voltage output

307 — Monitoring in real time whether tissue impedance value reach an end impedance threshold

308 — Monitoring in real time whether tissue impedance value is less than a holding impedance threshold within a predetermined time

309 — Issuing a prompt message if closure is successful and ending energy output

FIG 3

401     The first stage starts

402     Holding power $P_0$, output time $T_0$

403     Calculating initial impedance $Z_0$ and initial phase $\vartheta_0$

404     Calculating upper and lower impedance threshold values $Z_{lower}$ and $Z_{upper}$ based on the initial phase $\vartheta_0$

405     $Z_0 > Z_{lower}$ and $Z_0 < Z_{upper}$?

N

Y

406     No effective tissue clamp, prompting re-clamping tissue

407     Effective tissue clamp, entering the second stage

FIG 4

| 501 | The second stage starts |
| 502 | Calculating the power change trajectory based on the initial impedance |
| 503 | Adjusting output power according to the power change trajectory |
| 504 | Calculating average value of the tissue impedance change rate $Slope_{z_{avg}}$ |
| 505 | $\left|Slope_{z_{avg}}\right| < S_{hist}$ |
| 506 | Entering the third stage |

FIG 5

| 601 | The third stage starts |
| 602 | Calculating a target voltage value and an end impedance threshold |
| 603 | Adjusting linearly to a target voltage |
| 604 | Maintaining a constant voltage output |
| 605 | $Z > Z_{end}$? |
| 606 | Entering the fourth stage |

FIG 6

701 — The fourth stage starts

702 — Monitoring real-time tissue impedance values within a predetermined time

703 — $Z < Z_{hold}$?

N

Y

704 — Determining that the closure is successful

705 — Maintaining working status of the third stage

FIG 7

Output signal

time

FIG 8

FIG 9

## INTERNATIONAL SEARCH REPORT

| International application No. |
|---|
| **PCT/CN2022/124543** |

| A. | CLASSIFICATION OF SUBJECT MATTER |
|---|---|
| | A61B 18/14(2006.01)i; A61B 18/12(2006.01)i |

According to International Patent Classification (IPC) or to both national classification and IPC

| B. | FIELDS SEARCHED |
|---|---|

Minimum documentation searched (classification system followed by classification symbols)

    A61B; G06F

Documentation searched other than minimum documentation to the extent that such documents are included in the fields searched

Electronic data base consulted during the international search (name of data base and, where practicable, search terms used)

    CNTXT, ENTXTC, ENTXT, VEN, CJFD: 以诺康医疗科技, 姚龙洋, 王福源, 韦大纶, 刘振中, 骆威, 谐波, 高频, 采样, 相位差, 阻抗, 功率, 加权, 电压, 电流, 均值, 过零, FFT, 优化, 有效值, goertzel, harmonic, high frequency, sample, input, offset, phase, difference, impedance, power, weight, voltage, current, mean value, average, zero crossing, fast fourier transform algorithm, optimize, virtual value, effective, reference

| C. | DOCUMENTS CONSIDERED TO BE RELEVANT |
|---|---|

| Category* | Citation of document, with indication, where appropriate, of the relevant passages | Relevant to claim No. |
|---|---|---|
| PX | CN 115024814 A (YINUOKANG MEDICAL TECHNOLOGY (SUZHOU) CO., LTD.) 09 September 2022 (2022-09-09) <br>    claims 1-10 | 1-10 |
| PX | CN 114886552 A (YINUOKANG MEDICAL TECHNOLOGY (SUZHOU) CO., LTD.) 12 August 2022 (2022-08-12) <br>    description, paragraphs 119-213, and claims | 1-10 |
| X | CN 106132331 A (OLYMPUS CORP.) 16 November 2016 (2016-11-16) <br>    description, paragraphs 31-141 | 1, 7-10 |
| X | CN 105832406 A (ANJIN MEDICAL TECHNOLOGY (BEIJING) CO., LTD.) 10 August 2016 (2016-08-10) <br>    description, paragraphs 2-10 and 22-88 | 1, 7-10 |
| X | US 2008082098 A1 (TANAKA KAZUE et al.) 03 April 2008 (2008-04-03) <br>    description, paragraphs 19-48 | 1, 7-10 |
| X | JP 2007143878 A (OLYMPUS MEDICAL SYSTEMS CORP.) 14 June 2007 (2007-06-14) <br>    description, paragraphs 24-69 | 1, 7-10 |

☑ Further documents are listed in the continuation of Box C.     ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **10 January 2023** | **18 January 2023** |

| Name and mailing address of the ISA/CN | Authorized officer |
|---|---|
| **China National Intellectual Property Administration (ISA/CN)** <br> **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

## INTERNATIONAL SEARCH REPORT

International application No.

**PCT/CN2022/124543**

### C. DOCUMENTS CONSIDERED TO BE RELEVANT

| Category* | Citation of document, with indication, where appropriate, of the relevant passages | Relevant to claim No. |
|---|---|---|
| A | CN 105877836 A (HOCER BEIJING MEDICAL TECHNOLOGIES CO., LTD.) 24 August 2016 (2016-08-24)<br>entire document | 1-10 |
| A | US 2007173803 A1 (WHAM ROBERT H et al.) 26 July 2007 (2007-07-26)<br>entire document | 1-10 |

Form PCT/ISA/210 (second sheet) (January 2015)

**INTERNATIONAL SEARCH REPORT**
Information on patent family members

International application No.

**PCT/CN2022/124543**

| Patent document cited in search report | | | Publication date (day/month/year) | Patent family member(s) | | | Publication date (day/month/year) |
|---|---|---|---|---|---|---|---|
| CN | 115024814 | A | 09 September 2022 | None | | | |
| CN | 114886552 | A | 12 August 2022 | None | | | |
| CN | 106132331 | A | 16 November 2016 | JP | 5964000 | B1 | 03 August 2016 |
| | | | | WO | 2016031382 | A1 | 03 March 2016 |
| | | | | EP | 3108840 | A1 | 28 December 2016 |
| | | | | US | 2016367308 | A1 | 22 December 2016 |
| CN | 105832406 | A | 10 August 2016 | None | | | |
| US | 2008082098 | A1 | 03 April 2008 | EP | 1905371 | A1 | 02 April 2008 |
| JP | 2007143878 | A | 14 June 2007 | US | 2007123847 | A1 | 31 May 2007 |
| CN | 105877836 | A | 24 August 2016 | None | | | |
| US | 2007173803 | A1 | 26 July 2007 | AU | 2007200268 | A1 | 09 August 2007 |
| | | | | CA | 2574690 | A1 | 24 July 2007 |
| | | | | EP | 1810630 | A1 | 25 July 2007 |
| | | | | JP | 2012196458 | A | 18 October 2012 |
| | | | | JP | 2007195973 | A | 09 August 2007 |
| | | | | EP | 2298203 | A1 | 23 March 2011 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202210482325